# EUROPEAN PATENT SPECIFICATION

(11) **EP 1 036 162 B1**
(45) Date of publication and mention of the grant of the patent: **06.03.2013**
(21) Application number: 99946954.7
(22) Date of filing: 17.09.1999
(51) Int. Cl.: C12N 5/00, C12N 5/02, C12N 5/074, C12N 5/10

(54) **ENGRAFTABLE NEURAL STEM CELLS FOR BRAIN TUMOR THERAPY**
TRANSPLANTIERBARE NEURONALE STAMMZELLEN ZUR THERAPIE VON GEHIRNTUMOREN
CELLULES SOUCHES NEURONALES POUVANT ETRE GREFFEES POUR TRAITER LES TUMEURS AU CERVEAU

(30) Priority: 07.10.1998 US 168350
(43) Date of publication of application: 20.09.2000
(73) Proprietor: The Children's Medical Center Corporation, Boston, Massachusetts 02115 (US); Northeastern Ohio Universities College of Medicine, Rootstown, Ohio 44272-0095 (US); THE GENERAL HOSPITAL CORPORATION, Charlestown, MA 02129-2000 (US)
(72) Inventor: SNYDER, Evan, Y., Jamaica Plain, MA 02130 (US); LYNCH, William, P., Ravenna, OH 44266 (US); BREAKEFIELD, Xandra, O., Newton, MA 02159 (US); ABOODY, Karen, Needham, MA 02494 (US)
(74) Representative: WP Thompson
(86) International application number: PCT/US1999/021311
(87) International publication number: WO 2000/020560

(56) References cited:
- US-A- 5 750 376
- US-A- 5 753 506
- LYNCH WILLIAM P ET AL: "Late virus replication events in microglia are required for neurovirulent retrovirus-induced spongiform neurodegeneration: Evidence from neural progenitor-derived chimeric mouse brains" JOURNAL OF VIROLOGY, vol. 70, no. 12, 1996, pages 8896-8907, XP002305092 ISSN: 0022-538X
- PARK K I ET AL: "TRANSPLANTATION OF NEUROTROPHIN-3 (NT-3) EXPRESSING NEURAL STEM-LIKE CELLS INTO HYPOXIC-ISCHEMIC (III) BRAIN INJURY" ABSTRACTS OF THE SOCIETY FOR NEUROSCIENCE, SOCIETY FOR NEUROSCIENCE, WASHINGTON, DC, US, vol. 23, no. 1/2, 1997, page 346, XP009036839 ISSN: 0190-5295
- MARTINEZ-SERRANO A ET AL: "CNS-DERIVED NEURAL PROGENITOR CELLS FOR GENE TRANSFER OF NERVE GROWTH FACTOR TO THE ADULT RAT BRAIN: COMPLETE RESCUE OF AXOTOMIZED CHOLINERGIC NEURONS AFTER TRANSPLANTATION INTO THE SEPTUM" JOURNAL OF NEUROSCIENCE, NEW YORK, NY, US, vol. 15, no. 8, August 1995 (1995-08), pages 5668-5680, XP000867352 ISSN: 0270-6474
- GROVES A K ET AL: "Repair of demyelinated lesions by transplantation of purified O-2A progenitor cells" NATURE, MACMILLAN JOURNALS LTD. LONDON, GB, vol. 362, no. 6419, 1 April 1993 (1993-04-01), pages 453-455, XP002960450 ISSN: 0028-0836
- TAKAMIYA Y ET AL: "Gene therapy of malignant brain tumors: A rat glioma line bearing the herpes simplex virus type 1 thymidine kinase gene and wild type retrovirus kills other tumor cells" JOURNAL OF NEUROSCIENCE RESEARCH, vol. 33, no. 3, 1992, pages 493-503, XP008038515 ISSN: 0360-4012
- FROLICHSTHAL-SCHOELLER P ET AL: "Matrix metalloproteinases and tissue inhibitors of metalloproteinases in totipotent human embryonic neural stem cells. A possible model for glioma invasion" PROCEEDINGS OF THE AMERICAN ASSOCIATION FOR CANCER RESEARCH ANNUAL MEETING, vol. 39, March 1998 (1998-03), page 292, XP001203709 & 89TH ANNUAL MEETING OF THE AMERICAN ASSOCIATION FOR CANCER RESEARCH; NEW ORLEANS, LOUISIANA, USA; MARCH 28-APRIL 1, 1998 ISSN: 0197-016X
- ABOODY K S ET AL: "Neural progenitor/stem cells migrate throughout and express foreign genes within experimental gliomas: A potential gene therapy approach to brain tumors" PROCEEDINGS OF THE AMERICAN ASSOCIATION FOR CANCER RESEARCH ANNUAL MEETING, vol. 40, March 1999 (1999-03), page 88, XP001182943 & 90TH ANNUAL MEETING OF THE AMERICAN ASSOCIATION FOR CANCER RESEARCH; PHILADELPHIA, PENNSYLVANIA, USA; APRIL 10-14, 1999 ISSN: 0197-016X
- ABOODY KAREN S ET AL: "Neural stem cells display extensive tropism for pathology in adult brain: Evidence from intracranial gliomas" PROCEEDINGS OF THE NATIONAL ACADEMY OF SCIENCES OF THE UNITED STATES OF AMERICA, vol. 97, no. 23, 7 November 2000 (2000-11-07), pages 12846-12851, XP002305094 ISSN: 0027-8424
- FLAX ET AL.: 'Engraftable human neural stem cells respond to developmental cues, replace neurons and express foreign genes' NATURE BIOTECHNOLOGY. vol. 16, November 1998, pages 1033 - 1039, XP002922945
- SVENDSEN C.N.: 'Review Article: Neural stem cells for brain repair' ALZHEIMER'S RESEARCH. vol. 3, 04 August 1997, pages 131 - 135, XP002922946
- BARBA ET AL.: 'Thymidine Kinase-mediated killing of rat brain tumors' J. NEUROSURG., vol. 79, November 1993, pages 729 - 735, XP002922947
- TAKAMIYA ET AL.: 'An experimental model of retrovirus gene therapy for malignant brain tumors' J. NEUROSURG., vol. 79, July 1993, pages 104 - 110, XP002922948

## Description

### FIELD OF THE INVENTION

This invention is in the field of gene therapy, more particularly the field of using neuronal cells to treat brain tumors.

### Government Support

This invention was made with support from the NIH under grant number P20-HD18655, and the United States government has certain rights in this invention.

### BACKGROUND

An effective gene therapy for the treatment of brain tumors has been an elusive goal for many years. Glioblastoma multiforma, which is virtually untreatable, and the less malignant anaplastic astrocytoma account for about one-quarter of the 5,000 intracranial gliomas diagnosed yearly in the United States; 75 percent of gliomas in adults are of this category. Because of its profound and uniform morbidity, it contributes more to the cost of cancer on a per capita basis than does any other tumor. The patient, commonly stricken in the fifth decade of life, enters a cycle of repetitive hospitalizations and operations while experiencing the progressive complications associated with relatively ineffective treatments of radiation and chemotherapy ("Harrison's Principles of Internal Medicine," edited by Isselbacher, Braunwald, Wilson, Martin, Fauci and Kasper, 13th Edition, p. 2262, McGraw-Hill, Inc. 1994).

One of the impediments to gene therapy of brain tumors such as gliomas, has been the degree to which they expand, migrate widely and infiltrate normal tissue. Most gene therapy strategies to date are viral vector-based, yet extensive distributions of sufficient amounts of viral vector-mediated genes to large regions and numbers of cells typically in need has often been disappointingly limited. Interestingly, one of the defining features of normal neural progenitors and stem cells is their migratory quality. Neural stem cells (NSCs) are immature, uncommitted cells that exist in the developing, and even adult, CNS and postulated to give rise to the array of more specialized cells of the CNS. They are operationally defined by their ability to self-renew and to differentiate into cells of most (if not all) neuronal and glial lineages in multiple anatomical and development contexts, and to populate developing and/or degenerating CNS regions.¹⁻⁵

With the first recognition that neural cells with stem cell properties, reproduced in culture, could be reimplanted into mammalian brain where they could reintegrate appropriately and seamlessly in the neural architecture and stably express foreign genes⁶⁻⁷, gene therapists began to speculate how such a phenomenon might be harnessed for therapeutic purposes. These, and the studies which they spawned (reviewed elsewhere^{1-5,8}), provided hope that the use of neural progenitor/stem cells, by virtue of their inherent biology, might circumvent some of the present limitations of presently available gene transfer vehicles (e.g., non-neural cells, viral vectors, synthetic pumps), and provide the basis for a variety of novel therapeutic strategies.

Their use as graft material has been clearly illustrated by the prototypical neural progenitor clone, C17.2, a clone with which we have had extensive experience^{6,9-16,17} and which was used in the studies presented here. C17.2 is a mouse cell line from postnatal day 0 cerebellum immortalized by infection with a retroviral construct containing the avian *myc* gene. This line has been transduced to constitutively express the *lacZ* and *neo*R genes. When transplanted into germinal zones throughout the brain, these cells have been shown to migrate, cease dividing, and participate in the normal development of multiple regions at multiple stages (fetus to adult) along the murine neuraxis, differentiating appropriately into diverse neuronal and glial cell types as normal, non-tumorigenic cytoarchitectural constituents. They intermingle non-disruptively with endogenous neural progenitor/stem cells, responding to the same spatial and temporal cues in a similar manner. Crucial for therapeutic considerations, the structures to which C17.2 cells contribute develop and maintain neuroanatomical normality. In their earliest therapeutic use, they served to deliver a missing gene product throughout the brains of mice with a lysosomal deficiency state and cross-corrected host cells by release and uptake of a lysosomal enzyme⁹. The feasibility of a neural progenitor/stem cell-based strategy for the delivery of therapeutic molecules directly to and throughout the CNS was first affirmed by correcting the widespread neuropathology of a murine model of the genetic neurodegenerative lysosomal storage disease mucopolysaccaridosis type VII, caused by an inherited deletion of the β-glucuronidase (GUSB) gene, a condition that causes mental retardation and early death in humans. Exploiting their ability to engraft diffusely and become integral members of structures throughout the host CNS, GUSB-secreting NSCs were introduced at birth into subventricular germinal zones, and provided correction of lysosomal storage in neurons and glia throughout mutant brains. In so doing, it established that neural transplantation of neural progenitor cells could provide a novel therapeutic modality.

What is needed is a way to treat tumors which are diffuse, infiltrating and/or metastasizing. What is needed is a way to treat tumors locally to maximize the impact on the tumor and reduce the toxicity to the patient.

### SUMMARY OF THE INVENTION

The present invention relates to a genetically modified neural stem cell that contains a gene encoding a therapeutic agent for use in a method for the treatment of a brain tumor characterised in that said genetically modified neural stem cell is administered intracranially to the vicinity of a tumor cell present in a brain of a mammal to inhibit tumor growth.

The therapeutic agent may be a pro-drug activating enzyme or a suicide gene. The gene encoding the therapeutic agent may be encoded by a viral vector. The viral vector may be a replication defective retroviral vector. The viral vector may be a replication conditional herpes viral vector. The tumor cell may be a malignant glioma cell.

Any subject matter described which does not fall within the scope of the claims is provided for background information.

### BRIEF DESCRIPTION OF DRAWINGS

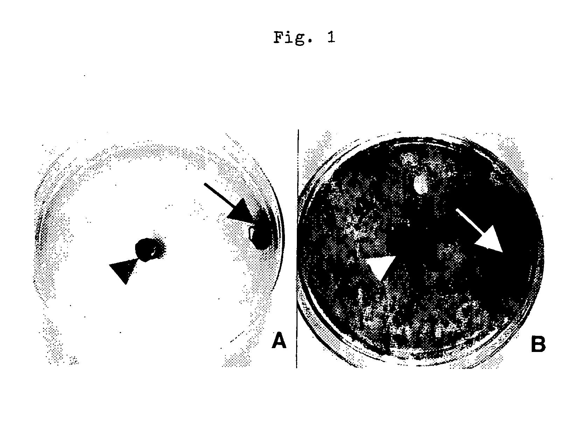

Figures 1A and 1B illustrate the migratory capacity of neural progenitor/step C 17.2 cell *in vitro.* After 5 days of incubation there was a wide distribution of C17.2 cells (Fig. 1B), suggesting that they had migrated far from their initial seeding in the cylinder, compared to TR-10 cells (Fig. 1A), which remained localized to the area of initial seeding in the cylinders. These patterns were observed whether the cells were plated directly on top of the glioma cells (right-sided cylinder [arrows] or simply in juxtaposition to them (center cylinder [arrows]).

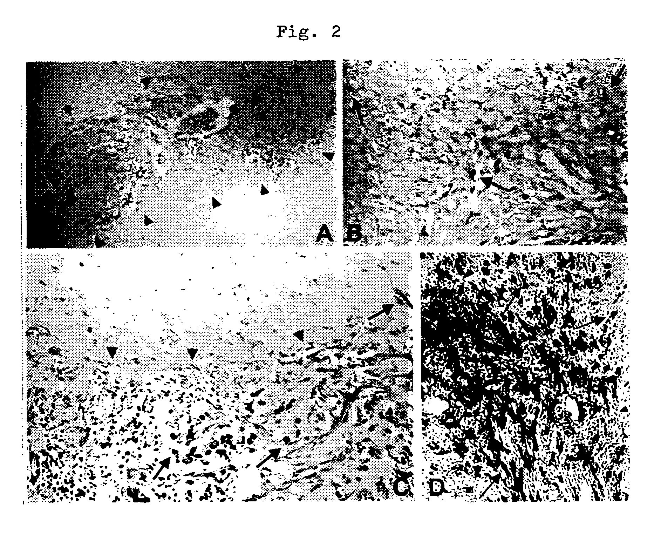

Figures 2A, 2B, 2C and 2D illustrate foreign gene-expressing neural progenitor/stem cells extensive migration throughout experimental tumor mass, and slightly beyond advancing tumor edge, appearing to "track" migrating tumor cells. (Fig. 2A) day 2 shown at 4X; arrowheads demarcate the approximate edges of tumor mass; (Fig. 2B) high power at 10X where Xgal, blue-staining NSCs [arrows] are interspersed between tumor cells staining dark red. (Fig. 2C) View of tumor mass 10 days after intra-tumoral injection showing Xgal+blue, C17.2 NSCs have infiltrated the tumor but largely stop at the edge of the darkly red stained tumor tissue with some migration into surrounding tissue when the blue-staining NSC appears to be "following" an invading, "escaping" cell [arrow] (10X). (Fig. 2D) CNS-1 tumor cells implanted into an adult nude mouse frontal cortex, there is extensive migration and distribution of blue C17.2 cells throughout the infiltrating experimental tumor bed, up to and along the infiltrating tumor edge [arrows], and where many tumor cells are invading normal tissue, into surrounding tissue in virtual juxtaposition to aggressive tumor cells [arrows] (10X).

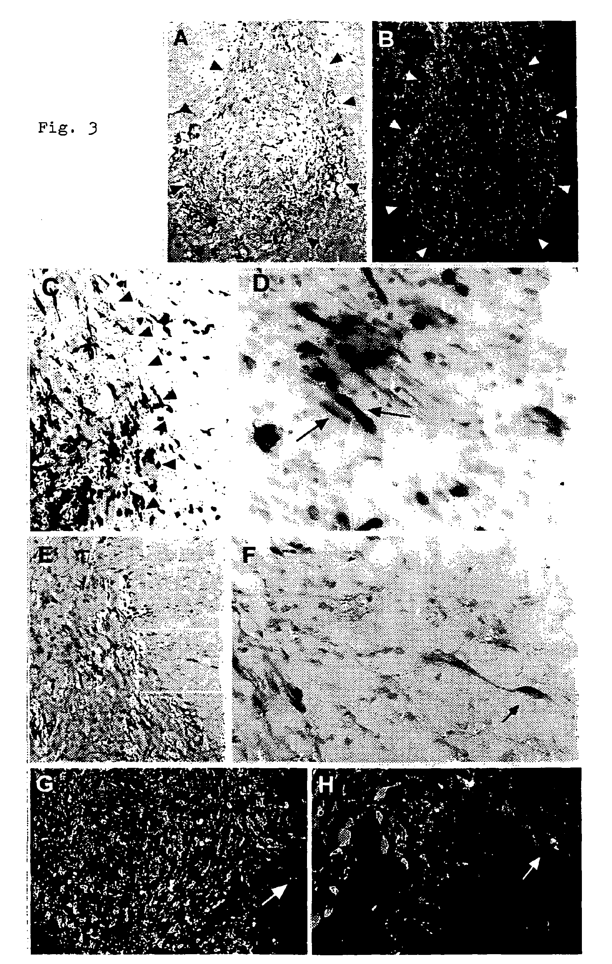

Figures 3A, 3B, 3C, 3D, 3E, 3F, 3G and 3H illustrate the neural progenitor/stem cells appearance to "track" migrating tumor cells away from main tumor mass; (Fig. 3A, 3B) parallel sections: low power C 17.2 cells distributed throughout tumor and surrounding edge [Fig. 3A) Xgal and neutral red, Fig. 3B) double immunofluorescent labelling with Texas red and FITC]; (Figs. 3C, 3D) low and high power of tumor edge and migrating tumor cell in juxtaposition to C17.2 cell (Xgal and neutral red); (Figs. 3G, 3H) low and high power of single migrating tumor cells in juxtaposition to C17.2 cells (double immunofluorescent labelling with texas red and FITC).

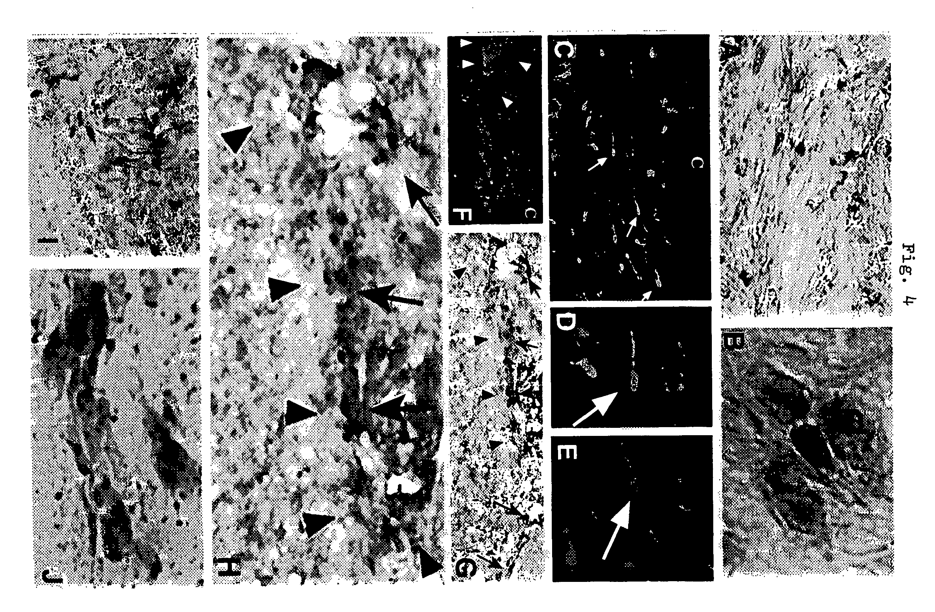

Figures 4A, 4B, 4C, 4D, 4E, 4F and 4G illustrate neural progenitor/stem cells implanted at distant site from main tumor bed migrating throughout normal tissue target CNS-1 tumor cells; (Figs. 4A, 4B) same hemisphere: 3x10⁴ CNS-1 tumor cells implanted into right frontal lobe. On day 6, 4x10⁴ C17.2 cells injected into right frontoparietal lobe (4mm caudal tumor injection). Animals sacrificed on day 12 (shown) and day 21, C17-2 cells seen in tumor bed (Xgal and neutral red). (Figs. 4C, 4D, 4E) Contralateral hemisphere: 3x10⁴ CNS-1 tumor cells implanted into left frontal lobe and 5x10⁴ CNS-1 tumor cells implanted into left frontoparietal lobe. On day 6, 8x10⁴ C17-2 cells were injected into right front lobe. Animals were sacrificed on day 12 and 21 (shown); c) 4x C17.2 cells (red) seen migrating towards tumor (green) from opposite side of the brain, d) 10x C17.2 cells (red) seen actively migrating across central commisure (double immunofluorescence), e) 20x C17-2 cells (blue) seen entering tumor (black arrows) (Xgal/neutral red). (Figs. 4F, 4G) Intraventricular: 5x10⁴ CNS-1 tumor cells were implanted into right frontal lobe. On day 6, 8x10⁴ C17.2 cells were injected into right or left (shown) lateral ventricle.

### DETAILED DESCRIPTION OF THE INVENTION

The experiments presented herein demonstrate that NSCs (prototypical clone C17.2) when implanted into an experimental glioma, will distribute throughout the tumor and migrate along with aggressively advancing tumor cells, while continuing to express their reporter gene lacZ. (One of the glioma lines used, astrocytoma cell line CNS-1, demonstrates single cell infiltration and invasive characteristics similar to those of human glioblastomas¹⁸). Furthermore, the neural progenitor/stem cells seem to migrate slightly beyond and surround the invading tumor border. In additional experiments, where neural progenitors were implanted at a distant site from the tumor bed, in the same hemisphere, opposite hemisphere, or lateral ventricle, they migrated through normal tissue moving specifically toward CNS-1 tumor cells. They were found to accumulate in or near the tumor bed as well as near or in direct juxtaposition to the individual infiltrating tumor cells.

Not wishing to be bound by any particular theory, the inventors propose that this neural progenitor/stem cell system migrate towards a trophic gradient of growth factors produced by the tumor cells. Thus, NSCs may provide a unique platform for the dissemination of therapeutic genes to the proximity of or into tumors that previously were inaccessible. These observations further suggest a number of other new gene therapy approaches. These may include the dissemination of cytotoxic gene products, but could also include factors that directly promote differentiation of neoplastic cells as well as the more efficacious delivery of viral vectors encoding therapeutic genes to be incorporated by tumor cells (e.g. suicide genes, differentiating agents, receptors to trophins). Because NSCs can be engineered to package and release replication-defective retroviral particles or replication-conditional herpes virus vectors which, in turn, may serve as vectors for the transfer of genes to CNS cells, neural progenitor/stem cells should serve to magnify the efficacy of viral-mediated gene delivery to large regions in the brain.

One effective mode of therapy for experimental brain tumors has been prodrug activation. Initially, prodrug activation enzymes were limited to antibodies directed against tumor enriched antigens. New strategies incorporate genes for these enzymes into viral vectors. Among the prodrug activating systems shown to be effective for gliomas *E.coli* cytosine deaminase (CD), HSV-TK and cytochrome p450 have been demonstrated to have a drug mediated bystander effect. Of these CD gives the best reported "bystander" effect. CD converts the nontoxic prodrug 5-fluorocytosine (5-FC) to 5-fluorouridine (5-FU) metabolites. 5-FU is a chemotherapeutic agent which has selective toxicity for actively dividing cells, thus primarily targeting tumor cells. In addition, 5-FU and its toxic metabolites can readily pass into adjacent and surrounding cells by non-facilitated diffusion. Brain tumors may require only a small number of cells expressing CD (about 2 % evenly distributed) to generate significant anti-tumor effects when treated with systemic, non-toxic levels of 5-FC. Our results support the hypothesis that transduced NSCs would disperse CD expression efficiently throughout the tumor and even "track" single migrating, "escaping" tumor cells.

Another approach to brain tumor gene therapy has been selective gene transfer to tumor cells in combination with pharmacotherapy, e.g., the HSV-TK gene, when transduced via retrovirus into a dividing population of brain tumor cells, confers a lethal sensitivity to the drug ganciclovir. Recent modifications of retroviral constructs to increase efficiency of infection and cell-specific targeting hold promise for enhancing the potency of this strategy. Again, through the "bystander-effect", tumor destruction is effective even when only a fraction of the cells express HSV-TK; adjacent tumor cells not expressing HSV-TK also appear to be eliminated. Attempts to improve efficiency of tumor destruction have focused on increasing the number of cells expressing the HSV-TK gene. The use of NSCs as packaging cells (which might then be self-eliminated) may prove to be an effective extended delivery system of the lethal gene to neighboring mitotic tumor cells, especially individual, infiltrating tumor cells.

In conclusion, genetically modified neural progenitor/stem cells have the potential to supply a range of tumor selective agents throughout mature and developing brains. The experiments presented here demonstrate the ability of NSCs: (1) to migrate/distribute quickly and effectively throughout the main tumor bed when implanted directly into the experimental gliomas; (2) to migrate beyond and "surround" (as if to contain) the invading tumor border; (3) to seemingly "track" individual, infiltrating tumor cells into surrounding tissue; (4) to migrate through normal tissue from distant sites to target CNS-1 tumor; and (5) to show stable expression of a foreign gene, in this case *lacZ,* throughout the tumor bed and in juxtaposition to tumor cells. These results lay the groundwork for future therapeutic brain tumor studies, providing critical support for the use of neural progenitor/stem cells as an effective delivery vehicle for tumor directed, vector-mediated enzyme/prodrug gene therapy.

### Other cells

The HCN-1 cell line is derived from parental cell lines from the cortical tissue of patients with unilateral megalencephaly growth (Ronnett G.V. et al., Science 248:603-5, 1990). HCN-1A cells have been induced to differentiate to a neuronal-like morphology and stain positively for neurofilament, neuron-specific enolase and p75NGFR, but not for myelin basic proteins, S-100 or glial fibrillary acidic protein (GFAP). Because these cells also stain positively for γ-amino butyric acid and glutamate, they appear to become neuro-transmitting bodies. Earlier Poltorak M. et al. (Cell Transplant 1(1):3-15, 1992) observed that HCN-1 cells survived in the brain parenchyma and proposed that these cells may be suitable for intracerebral transplantation in humans.

Ronnett GV et al. (Neuroscience 63(4): 1081-99, 1994) reported that HCN-1 cells grew processes resembling neurons when exposed to nerve growth factor, dibutyryl cyclic AMP and isobutylmethylxanthine.

The nerve cells also can be administered with macrophages which have been activated by exposure to peripheral nerve cells. Such activated macrophages have been shown to clean up the site of CNS trauma, for example a severed optic nerve, after which new nerve extensions started to grow across the lesion. Implanting macrophages exposed to CNS tissue (which secretes a chemical to inhibit macrophages) or nothing at all resulted in little or no regeneration (Lazarov-Spiegler et al., FASEB J. 10:1, 1996).

Fetal pig cells have been implanted into patients with neurodegenerative diseases, such as Parkinson's disease and Huntington's chorea, and intractable seizures, in whom surgical removal of the excited area would otherwise have been performed. Such cells, if properly screened for retroviruses, could also be used in the inventive method.

### Other cytokines, growth factors and drugs

Certain cytokines, growth factors and drugs are optionally used in the transplant area or may be administered concomitantly with the transplant.

Known cytokines include interleukins (IL) IL-1, IL-2, IL-3, IL-4, IL-5, IL-6, IL-7, IL-8, IL-10, and IL-11; tissue necrosis factors TNF, TNFα, also lymphotoxin (LT) and TNFβ; interferons (IFN) IFNα, IFNβ and IFNγ; and tissue growth factor (TGF). The colony-stimulating factors (CSFs) are specific glycoproteins that are thought to be involved in the production, differentiation and function of stem cells.

Nerve growth factor (NGF) has been shown to increase the rate of recovery in spatial alternation tasks after entorhinal lesions, possibly by acting on cholinergic pathways (Stein and Will, Brain Res. 261: 127-31, 1983).

### EXAMPLES

### Experimental Methods

### Cells:

C17-2 and TR-10 cells were maintained in Dulbecco's modified Eagle's medium (DMEM; Mediatech, Washington, DC) supplemented with 10% fetal calf serum (FCS; Sigma, St. Louis, MO), 5% horse serum (HS; Gibco), 1% Glutamine (2mM; Gibco), 1% penicillin/streptomycin (Sigma). CNS-1 cells were stably transduced with the PGK-GFP-IRES-NeoR retroviral vector construct to express green fluorescent protein (GFP) as previously described (ref. Aboody-Guterman et al., 1997), and maintained in RPMI-1640 (Bio Whittaker) supplemented with 10% FCS and 1% penicillin/streptomycin (Sigma). Cell structure studies were performed in 100mm petri dishes under standard conditions: humidified, 37°C, 5% CO₂ incubator. *In vitro* studies; CNS-1 glioma cells were plated to approximately 60-70% confluency around a 5mm cylinder (i.e., free of CNS-1 cells) into which 40,000 C17.2 or TR-10 cells plated overnight. At the same time, 40,000 C17.2 or TR-10 cells were placed into a 5mm cylinder placed directly on top of adhered CNS-1 cells. The next day, cylinders were removed and plates rinsed well with PBS to remove any floating cells, media was replaced, and plates were incubated for 5 days. Plates were subsequently stained for β-galactosidase overnight after .5% glutaraldehyde fixation. (Note: both C17.2 and TR-10 cells are >90% blue with X-gal staining.) *In vivo* studies; 48 hours prior to transplant, C17.2 and TR-10 cells were incubated with BUdR (Sigma) at a concentration of 10µM. Plated cells were rinsed with PBS, trypsinized, resuspended in media and counted on the Coulter counter. Desired number of cells were spun down at 4°C in the centrifuge for 4 minutes and 1100 rpm to obtain a pellet. Media was removed; cells were rinsed by resuspending in PBS and respun. PBS was removed and the appropriate amount of PBS added to resuspend cells at final desired concentration. Cells were kept on ice, and gently triturated prior to each animal injection. Cells not labelled with BUdR were prepared for injection in similar manner.

### Animals:

Animal studies were performed in accordance with guidelines issued by the Massachusetts General Hospital Subcommittee on Animal Care. Animals used: adult CD-Fisher rats (Charles River) and 8-10 week old adult, approximately 20 gram female nude mice (random bred Swiss white obtained from Cox 7, MGH-East).

### Surgery and Sacrifice:

Animals were anesthetized by an i.p. injection of .15 ml of 20% ketamine HCL (KETALAR 100 mg/ml; Parke-Davis, Morris Plains, NJ), 20% xylazine (ROMPUN 20 mg/ml; Miles Inc., Shawnee Mission, KS), 60% sodium chloride (0.9%; Abbott Laboratories, North Chicago, IL) and immobilized in stereotactic apparatus (Kopf, Tujunga, CA). Intracerebral injections were stereotactically performed by making a linear scalpel skin incision on top of the skull. A burr hole was drilled into the skull with a high speed drill 2mm lateral to the bregma on the coronal suture. After incising the dura with a sterile needle and obtaining hemostasis, desired number of tumor cells suspended in 1 µl of 1X Dulbecco's phosphate-buffered salt solution (PBS pH 7.4; Mediatech, Herndon, VA) were injected with a 26 gauge 5 µl Hamilton syringe to specified location (see protocols below) over a 3 to 5 minute period. After retracting the needle over a 2-4 minute period, bone-wax (Ethicon, Somerville, NJ) was used to occlude the burr hole, betadine applied to surgical area, and the skin sutured closed. Animals receiving a second injection at a later date were anesthetized, immobilized in stereotactic apparatus, and cells injected as per specific protocol (see below). Animals were sacrificed on stated days with an overdose of anesthesia and subsequent intracardiac perfusion with PBS followed by 4% paraformaldehyde+2mM MgCl₂ (pH 7.4). Brains were removed and post-fixed overnight at 4°C and then transferred to 30% sucrose in PBS + 2mM MgCl₂ (pH 7.4) for 3-7 days to cyroprotect. Brains were stored at -80°C and then 10-15 micron coronal serial sections were cut to cyrostat (Leica CM 3000).

### BUdR labelling of engrafted C17-2 cells:

Selected animals received 3 intraperitoneal injections of 1 ml/100g body weight 20µM BUdR stock solution (Sigma) over 24 hours prior to sacrifice (.2 ml/injection per 20g mouse).

### Histopathological and Immunohistochemical Studies:

Tissue sections were stained with (1) Xgal and counterstained with neutral red (2) hematoxylin and eosin (3), double immunofluorescent labelling was performed with Texas red anti-beta-galactosidase and FITC anti-GFP. Slides were examined with light microscopy, fluorescent microscopy. CNS-1 tumor cells were also examined without staining under confocal fluorescent microscopy.

### Example 1. Migratory Capacity of NSCs in Culture

To determine properties of the NSCs in association with glioma cells, studies were initially performed in culture comparing the relative migratory capacity of NSCs (clone C17.2) to fibroblasts (the lacZ-expressing TR-10 fibroblast cell line) when co-cultured with glioma cells. C17.2 and TR-10 cells were maintained in Dulbecco's modified Eagle's medium (DMEM; Mediatech, Washington, DC) supplemented with 10% fetal calf serum (FCS; Sigma, St. Louis, MO), 5% horse serum (HS; Gibco), 1% Glutamine (2mM; Gibco), 1% penicillin/streptomycin (Sigma). CNS-1 cells were stably transduced with the PGK-GFP-IRES-NeoR retroviral vector construct to express green fluorescent protein (GFP) as previously described (ref. Aboody-Guterman et al., 1997), and maintained in RPMI-1640 (Bio Whittaker) supplemented with 10% FCS and 1% penicillin/streptomycin (Sigma). Cell structure studies were performed in 100mm petri dishes under standard conditions: humidified, 37°C, 5% CO₂ incubator. CNS-1 glioma cells were plated to approx. 60-70% confluency around a 5mm cylinder (i.e., free of CNS-1 cells) into which 40,000 C17.2 or TR-10 cells plated overnight. At the same time, 40,000 C17.2 or TR-10 cells were placed into a 5mm cylinder placed directly on top of adhered CNS-1 cells. The next day, cylinders were removed and plates rinsed well with PBS to remove any floating cells, media was replaced, and plates incubated for 5 days. Plates were subsequently stained for β-galactosidase overnight after .5% glutaraldehyde fixation. (Note: both C17.2 and TR-10 cells are >90% blue with X-gal staining.)

There was a wide distribution of C17.2 cells (Fig. 1B), suggesting that they had migrated far from their initial sites in the cylinder, compared to the TR-10 cells (Fig. 1A), which remained localized to the area of initial seeding in the cylinder. These patterns were observed whether the cells were plated directly on top of the glioma cells (right-sided cylinder [arrows]) or simply in juxtaposition to them (center cylinder [arrows]).

### Example 2. Transgene-Expressing NSCs Migrate Throughout and Beyond Invading Tumor Mass in vivo

To determine the behavior of clone C17.2 NSCs introduced into brain tumors, experimental animals (syngeneic adult rats) first received an implant of 4 x 10⁴ D74 rat glioma cells in 1 µl injected into the right frontal lobe. Four days later, 1 x 10⁵ C17.2 NSCs in 1.5 µl PBS were injected at same coordinates directly into the D74 tumor bed. Animals were then sacrificed at days 2, 6, and 10 post-intratumoral injection and cyrostat sections of the brains were processed with Xgal histochemistry for β-galactosidase (βgal) activity to detect donor-derived cells and counterstained with neutral red to detect tumor cells.

Donor C17.2 NSCs were found extensively dispersed throughout the tumor within a few days, spanning an -8mm width of tumor as rapidly as 2 days after injection (Figs. 2A, 2B). This is a much more extensive and rapid dispersion compared to previous reports of 3T3 fibroblasts grafted into an experimental brain tumor³³. By day 10, C17.2 cells were seen throughout a majority of the tumor, clearly along the infiltrating tumor edge and slightly beyond it, drawn somewhat by the degenerative environment, seeming to "track" migrating tumor cells (Figs. 2C, 2D). C17.2 cells themselves did not become tumorigenic.

(Fig. 2A) Day 2 shown at 4X; arrowheads demarcate the approximate edges of tumor mass; even at lower power, the tumor can be seen to be intermixed with blue NSCs [arrows]. This is appreciated more dramatically at high power in (Fig. 2B) at 10X where Xgal+, blue-staining NSCs [arrow] are interspersed between tumor cells staining dark red. (Fig. 2C) This view of the tumor mass, 10 days after intra-tumoral injection nicely shows that Xgal+blue, C17.2 NSCs have infiltrated the tumor but largely stop at the edge of the darkly red stained tumor tissue (border indicated by arrowheads) with some migration into surrounding tissue when blue-staining NSC appears to be "following" and invading, "escaping" tumor cell [arrow] (10X). This phenomenon becomes even more dramatic when examining the behavior of C17.2 NSCs in an even more virulent, invasive and aggressive tumor than D74, the experimental CNS-1 astrocytoma in the brain of a nude mouse (Fig. 2D). CNS-1 tumor cells were implanted into an adult nude mouse frontal cortex (day 0). On day 6, 4 x 10⁴ C17.2 cells were implanted directly into the tumor bed. The animal pictured in (Fig. 2D) was sacrificed on day 12 post-tumor implantation, 6 days post-intra-tumoral injection. The cyrostat section pictured was processed with Xgal histochemistry for β-galactosidase activity to detect blue C17.2 NSCs and counterstained with neutral red to show dark red tumor cells. There is extensive migration and distribution of blue C 17.2 cells throughout the infiltrating experimental tumor bed, up to and along the infiltrating tumor edge [white arrows], and, where many tumor cells are invading normal tissue, into surrounding tissue in virtual juxtaposition to aggressive tumor cells [arrows] (10X).

### Example 3. NSCs "Track" Infiltrating Tumor Cells

CNS-1 tumor cells were labelled by retroviral transduction with green fluorescent protein (GFP), prior to implantation, to better distinguish single cells away from the main tumor bed¹⁷. GFP-expressing CNS-1 glioma cells (3 x 10⁴) in 1 µl PBS injected into right frontal lobe at stereotaxic coordinates 2mm lateral to bregma, on coronal suture, 3mm depth from dura. 4 x 10⁴ C17.2 or TR-10 cells in 1 µl PBS injected at same coordinates directly into tumor bed on day 6. 3-4 C17.2 animals (2 BUdR labelled, 1 BUdR pulsed) and 1-2 TR-10 control animals (1 BUdR labelled). Animals were sacrificed on days 9, 12, 16 and 21 post-tumor implantation. Cyrostat sectioned, fixed brain tissue was stained either with β-galactosidase (C17.2 cells blue) and neutral red (tumor cells dark red) or double immunofluorescence with Texas red anti-β-galactosidase (C17.2 cells red) and FITC anti-GFP (tumor cells green).

(Figs. 3A, 3B) parallel sections: low power of C17.2 cells distributed throughout tumor and surrounding edge [Fig. 3A) Xgal and neutral red, Fig. 3B) double immunofluorescent labelling with Texas red and FITC].

(Figs. 3C, 3D) low and high power of single migrating tumor cell in juxtaposition to C17.2 cell (Xgal and neutral red).

(Figs. 3E, 3F) low and high power of single migrating tumor cell in juxtaposition to C17.2 cell (Xgal and neutral red).

(Figs. 3G, 3H) low and high power of single migrating tumor cells in juxtaposition to C17.2 cells (double immunofluorescent labelling with Texas red and FITC).

### Example 4. NSCs Implanted at Distant Site Migrate Toward Tumor

To examine the capacity of NSCs to migrate through normal tissue and specifically target tumor cells, donor NSCs were injected into uninvolved sites distant from the main tumor bed in three separate paradigms, into the same hemisphere, into the opposite hemisphere, or into the lateral ventricles.

Same hemisphere: CNS-1 glioma cells (3 x 10⁴) in 1 µl PBS was injected into the right frontal lobe at stereotaxic coordinates 2mm lateral to bregma, on coronal suture, 3mm depth from dura. 4 x 10⁴ C17.2 or TR-10 cells in 1 µl PBS injected into right frontal parietal lobe at stereotaxic coordinates 3mm lateral and 4mm caudal to bregma, 3mm depth from dura on day 6. Two animals were sacrificed at days 12 and 21. At all time points, NSCs were found distributed within the main tumor bed as well as in juxtaposition to migrating tumor cells in surrounding tissue (Figs. 4A, 4B).

Apposite hemisphere: 3 x 10⁴ CNS-1 tumor cells in 1 µl PBS injected into left frontal lobe at stereotaxic coordinates 2mm lateral to bregma, on coronal suture, 3mm depths from dura, 5 x 10⁴ CNS-1 tumor cells in 1 µl PBS injected into left frontoparietal lobe 3 mm lateral and 4mm caudal to bregma, 3mm depth from dura, 8 x 10⁴ C17.2 cells in 2 µl PBS injected into right frontal lobe 2mm lateral and 2mm caudal to bregma, 3mm depth from dura on day 6. Two animals were sacrificed on day 12 and 21. (control - no tumor Coordinates: 2mm R of bregma, 2mm caudal, 3mm deep). NSCs were seen actively migrating across the central commissure towards the tumor on the opposite side of the brain, and then entering the tumor (Figs. 4C, 4D, 4E).

### Implantation away from CNS-1 tumor bed (intraventricular):

In this final paradigm 5 x 10⁴ CNS-1 tumor cells in 1 µl PBS was injected into the right frontal lobe 2mm lateral to bregma, on coronal suture, 3mm depth from dura. 8 x 10° C17.2 cells in 2 µl PBS injected into left or right ventricle 1mm lateral and 3mm caudal to bregma, 2mm depth from dura on day 6. Two animals were sacrificed on days 12 and 21. NSCs again were seen within the main tumor bed, as well as in juxtaposition to migrating tumor cells (Figs. 4F, 4G).

In each case, donor NSCs were found to migrate through normal tissue and "target" the tumor.

### ACKNOWLEDGEMENTS

### References

1. Gage FH, Ray J, Fisher LJ: Isolation, characterization and use of stem cells from the CNS. Ann Rev Neurosci 18:159-92 (1995).
2. Whittemore SR, Snyder EY: The physiologic relevance & functional potential of central nervous system-derived cell lines. Molecular Neurobiology 12(1):13-38 (1996).
3. McKay R: Stem cells in the central nervous system. Science 276:66-71 (1997).
4. Gage FH, Christen Y, (eds): Research & Perspectives in Neurosciences: Isolation, Characterization, & Utilization of CNS Stem Cells. Springer-Verlag, Heidelberg, Berlin (1997).
5. Snyder EY, Macklis JD: Neural cells with stem-like features may hold potential for CNS gene therapy & repair. N Engl J Med (submitted).
6. Snyder EY, Deitcher DL, Walsh C, et al: Multipotent neural cell lines can engraft & participate in development of mouse cerebellum. Cell 68:33-51 (1992).
7. Renfranz PJ, Cunningham MG, McKay RDG: Region-specific differentiation of the hippocampal stem cell line HiB5 upon implantation into the developing mammalian brain. Cell 66:713-729 (1991).
8. Snyder EY, Fisher LJ: Gene therapy for neurologic diseases. Current Opin. in Pediatrics 8:558-568 (1996).
9. Snyder EY, Taylor RM, Wolfe JH: Neural progenitor cell engraftment corrects lysosomal storage throughout the MPS VII mouse brain. Nature 374:367-370 (1995).
10. Lacorazza HD, Flax JD, Snyder EY, Jendoubi M: Expression of human β-hexosaminidase α-subunit gene (the gene defect of Tay-Sachs disease) in mouse brains upon engraftment of transduced progenitor cells. Nature Medicine 4:424-429 (1996).
11. Lynch WP, Snyder EY, Qualtierre L, Portis JL, Sharpe AH: Neither neurovirulent retroviral envelope protein nor viral particles are sufficient for the induction of acute spongiform neurodegeneration: evidence from engineered neural progenitor-derived chimeric mouse brains. Journal of Virology 70:8896-8907 (1996).
12. Snyder EY, Macklis JD: Multipotent neural progenitor or stem-like cells may be uniquely suited for therapy of some neurodegenerative conditions. Clinical Neuroscience 3: 310-316 (1996).
13. Snyder EY, Yoon CH, Flax JD, Macklis JD: Multipotent neural progenitors can differentiate towards the replacement of neurons undergoing targeted apoptopic degeneration in adult mouse neocortex. Proc Natl Acad Sci 94:11663-8 (1997).
14. Park KI, Jensen FE, Stieg PE, Snyder EY: Acute CNS injury may direct the migration, proliferation, & differentiation of neural stem cells: Evidence from the effect of hypoxia-ischemia on "reporter" cells. Soc Neurosci Abstr 21:2027 (1995).
15. Park KI, Jensen FE, Stieg PE, Himes T, Fisher I, Snyder EY: Transplantation of neurotrophin-3 (NT-3) expressing neural stem-like cells into hypoxic-ischemic (HI) brain injury. Soc Neurosci Abstr 23:346 (1997).
16. Park KI, Liu S, Flax JD, Nissim S, Stieg PE, Snyder EY: Transplantation of neural progenitor & stem-like cells: Developmental insights may suggest new therapies for spinal cord and other CNS dysfunction. Journal of Neurotrauma (in press).
17. Aboody-Guterman KS, Pechan PA, Rainov NG, Sena-Esteves M, Snyder EY, Wild P, Schraner E, Tobler K. Breakefield XO and Fraefel C: Green fluorescent protein as a reporter for retrovirus and helper virus-free HSV-1 amplicon vector-mediated gene transfer into neural cells in culture and in vivo. NeuroReport Vol. 8:3801-8 (1997).
18. Kruse CA, Molleston M, Parks EP, Schiltz PM, Kleinschmidt-DeMasters BK, and Hickey WF: A rat glioma model, CNS-1, with invasive characteristics similar to those of human gliomas: A comparison to 9L gliosarcoma. J of Neuro-Oncology 22:191-200 (1994).
19. Short et al.
20. Tamija T, Wei MX, Chase M, Ono Y, Lee F, Breakefield XO, and Chiocca EA: Transgene inheritance and retroviral infection contribute to the efficiency of gene expression in solid tumors inoculated with retroviral vector producer cells. Gene Therapy 2:531-538 (1995).
21. Deonariain MP, Spooner RA, and Epenetos AA: Genetic delivery of enzymes for cancer therapy. Gene Therapy 2:235-244 (1995).
22. Kramm CM, Sena-Esteves M, Barnett F, Rainov N, Schuback D, Yu J, Pechan P, Paulus W, Chiccoa EA, and Breakefield XO: Gene therapy for brain tumors. Brain Pathology 5:345-381 (1995).
23. Philpott GW, Shearer WT, Bower RW and Parker CW: Selective cytotoxicity of hepten-substituted cells with an antibody-enzyme conjugate. J Immunol. 111:921-929 (1973).
24. Huber BE, Austin EA, Richards CA, Davis ST and Good S: Metabolism of 5-fluorocytosine to 5-fluorouracil in human colorectal tumor cells transducted with the cytosine deaminase gene: Significant antitumor effects when only a small percentage of tumor cells express cytosine deaminase. Proc. Natl. Acad. Sci. 91:8302-8306 (1994).
25. Mullen C, Kilstrup M, and Blaese RM: Transfer of the bacterial gene for cytosine deaminase to mammalian cells confers lethal sensitivity to 5-fluorocytosine: a negative selection system. Proc. Natl. Sci. USA 89:33-37 (1992).
26. Kun Le, Gaijar A, Muhlbauer M et al: Clinical protocol: stereotactic injection of herpes simplex thymidine kinase vector producer cells (PA317-G1Tk1SvNa.7) and intravenous gangciclovir for the treatment of progressive or recurrent primary supratentorial pediatric malignant brain tumors. Hum Gene Ther 6:1231-1255 (1995).
27. Walther W, Stein U, Pfeil D: Gene transfer of human TNFα into glioblastoma cells permits modulation of mdr1 expression and potentiation of chemosensitivity. Int J Cancer 61:832-839 (1995).
28. Manome Y, Wen PY, Dong Y et al: Viral vector transduction of the human deoxycytidine kinase cDNA sensitizes glioma cells to the cytotoxic effects of cytosin arabinoside in vitro and in vivo. Nat Med 2:567-573 (1996).
29. Takamiya Y, Short MP, Moolten F, Fleet C, Mineta T, Breakefield XO, Martuza RL: An experimental model of retroviral gene therapy for malignant brain tumors. J Neurosurg 79:104-110 (1993).
30. Barba D, Hardin J, Ray J, Gage FH: Thymidine kinase-mediated killing of rat brain tumors. J Neurosurg 79:729-735 (1993).
31. Vrionis FD, Wu JK, Qi P, Cano WG, Cherington V: Tumor cells expressing the herpes simplex virus-thymidine kinase gene in the treatment of Walker 256 meningal neoplasia in rats. J Neurosurg 84:250-257 (1996).
32. Lyons RM, Forry-Schaudies S, Otto E, et al: An improved retroviral vector encoding the herpes simplex thymidine kinase gene increases antitumor efficacy in vivo. Cancer Gene Ther 2:273-280 (1995).
33. Rainov NG Kramm CM, Aboody-Guterman K, Chase M, Ueki K, Louis D, Harsh G, Chiocca EA, and Breakefield XO: Retrovirus-mediated gene therapy of experimental brain neoplasms using the herpes simplex virus-thymidine kinase/ganciclovir paradigm. Cancer Gene Therapy Vol. 3:2:99-106 (1996).
34. Somia N, Zoppe M, Verma IM: Generation of targeted retroviral vectors by single-chain variable fragment: An approach to in vivo gene delivery. Proc Natl Acad Sci USA 92:7570-7574 (1995).
35. Chen C, Chang Y, Ryan P et al: Effect of herpes simplex virus thymidine kinase expression level on gangciclovir-mediated cytotoxicity and the "bystander-effect". Hum Gene Ther 6:1467-1476 (1995).
36. Weinstein DE et al: C17 inhibits the proliferation of astrocytes & astrocytoma cells by a contact-mediated mechanism. Glia 3:130-139 (1990).

## Claims

1. A genetically modified neural stem cell that contains a gene encoding a therapeutic agent for use in a method for the treatment of a brain tumor **characterised in that** said genetically modified neural stem cell is administered intracranially to the vicinity of a tumor cell present in a brain of a mammal to inhibit tumor growth.

2. The genetically modified neural stem cell of Claim 1, wherein the therapeutic agent is a pro-drug activating enzyme.

3. The genetically modified neural stem cell of Claim 1, wherein the therapeutic agent is a suicide gene.

4. The genetically modified neural stem cell of Claim 1, wherein the gene encoding the therapeutic agent is encoded by a viral vector.

5. The genetically modified neural stem cell of Claim 4, wherein the viral vector is a replication defective retroviral vector.

6. The genetically modified neural stem cell of Claim 4, wherein the viral vector is a replication conditional herpes viral vector.

7. The genetically modified neural stem cell of Claim 1, wherein the tumor cell is a malignant glioma cell.

## Patentansprüche

1. Genetisch veränderte neuronale Stammzelle, die ein Gen enthält, dass einen therapeutischen Wirkstoff kodiert, zur Verwendung in eine Verfahren zur Behandlung eines Gehirntumors, **dadurch gekennzeichnet, dass** die genetisch veränderte neuronale Stammzelle intrakraniell in der Nähe einer Gehirn eines Säugetiers vorhandene Tumorzelle verabreicht wird, um den Tumorwachstum zu hemmen.

2. Genetisch veränderte neuronale Stammzelle nach Anspruch 1, wobei der therapeutische Wirkstoff ein Prodrug-aktivierendes Enzym ist.

3. Genetisch veränderte neuronale Stammzelle nach Anspruch 1, wobei der therapeutische Wirkstoff ein Suizid-Gen ist.

4. Genetisch veränderte neuronale Stammzelle nach Anspruch 1, wobei das Gen, das den therapeutischen Wirkstoff kodiert durch einen viralen Vektor kodiert ist.

5. Genetisch veränderte neuronale Stammzelle nach Anspruch 4, wobei der virale Vektor ein replikationsdefekter retroviraler Vektor ist.

6. Genetisch veränderte neuronale Stammzelle nach Anspruch 4, wobei der virale Vektor ein konditional replikativer herpesviraler Vektor ist.

7. Genetisch veränderte neuronale Stammzelle nach Anspruch 1, wobei die Tumorzelle eine maligne Gliomzelle ist.

## Revendications

1. Cellule souche neurale génétiquement modifiée qui contient un gène codant un agent thérapeutique destinée à être utilisée dans un procédé pour le traitement d'une tumeur cérébrale **caractérisée en ce que** ladite cellule souche neurale génétiquement modifiée est administrée par voie intracrânienne au voisinage d'une cellule tumorale présente dans un cerveau d'un mammifère pour inhiber la croissance morale.

2. Cellule souche neurale génétiquement modifiée selon la revendication 1 où l'agent thérapeutique est une enzyme activant un promédicament.

3. Cellule souche neurale génétiquement modifiée selon la revendication 1 où l'agent thérapeutique est un gène suicide.

4. Cellule souche neurale génétiquement modifiée selon la revendication 1 où le gène codant l'agent thérapeutique est codé par un vecteur viral.

5. Cellule souche neurale génétiquement modifiée selon la revendication 4 où le vecteur viral est un vecteur rétroviral défectif pour la réplication.

6. Cellule souche neurale génétiquement modifiée selon la revendication 4 où le vecteur viral est un vecteur herpès viral conditionnel pour la réplication.

7. Cellule souche neurale génétiquement modifiée selon la revendication 1 où la cellule tumorale est une cellule de gliome malin.
